# EUROPEAN PATENT APPLICATION

(11) **EP 4 066 779 A2**
(43) Date of publication of application: **05.10.2022**
(21) Application number: 20906021.9
(22) Date of filing: 24.12.2020
(51) Int. Cl.: A61C 9/00, A61C 13/34, A61C 13/00, A61C 19/04

(54) **METHOD FOR MATCHING STRUCTURE DATA AND SYSTEM FOR MATCHING STRUCTURE DATA USING SAME**

(30) Priority: 26.12.2019 KR 20190175289
(71) Applicant: Medit Corp., Seongbuk-gu Seoul 02855 (KR)
(72) Inventor: SONG, Myoung Woo, Seoul 06518 (KR)
(74) Representative: BCKIP
(86) International application number: PCT/KR2020/019143
(87) International publication number: WO 2021/133120

(57) **Abstract**

In a method for matching structure data according to the present disclosure, scanned data is used for a prepared part and library model data loaded in advance in a library is used for an unprepared part, such that the method has an advantage of improving reliability of oral model data required for an implant crown procedure.

## Description

### [Technical Field]

The present disclosure relates to a method for matching structure data and a matching system using the same, and more specifically, to a method for acquiring three-dimensional structure model data by combining three-dimensional library model data with three-dimensional scan model data acquired by a scan, and a system using the same.

### [Background Art]

It is important for humans to use all permanent teeth in a healthy way when using the permanent teeth, but in some cases, humans may perform mastication activities or the like by removing teeth damaged due to various problems such as tooth decay and taking an implant crown procedure to substitute the damaged teeth with artificial teeth.

When taking a crown procedure or an implant procedure, the patient extracts the existing tooth positioned at the corresponding procedure site, receives nerve treatment together with the tooth extraction, and has an artificial tooth implanted thereon.

Meanwhile, to manufacture a prosthesis applied to the patient for implanting the artificial teeth, a patient tooth model with a high level of precision is required, and the prosthesis is conventionally manufactured and processed based on an oral plaster acquired by taking impressions using a material such as alginate. However, recently, with the development of three-dimensional scanning and CAD/CAM fields, it is possible to provide a more suitable prosthetic treatment for each patient by scanning the patient's oral cavity through a three-dimensional scanner.

### [Summary of Invention]

### [Technical Problem]

A method for matching structure data according to the present disclosure provides a method for disposing three-dimensional library model data equipped on a library on three-dimensional oral model data, and allowing a portion corresponding to an upper portion of the three-dimensional library model to be aligned and merged by generating three-dimensional scan model data through a three-dimensional scanner or the like.

In addition, a system for matching structure data according to the present disclosure provides a matching system for loading three-dimensional library model data from a storage unit to dispose the three-dimensional library model data on oral model data, and removing and then allowing some areas of the three-dimensional library model to be aligned and merged by generating three-dimensional scan model data through a scan unit.

The objects of the present disclosure are not limited to the above-described objects, and other objects not mentioned will be clearly understood to those skilled in the art from the following descriptions.

### [Solution to Problem]

A method for matching structure data according to the present disclosure may include a library loading operation of loading a library of a structure on a user interface, a selecting operation of selecting a three-dimensional library model from the loading operation by a user, a library model setting operation of disposing the three-dimensional library model selected in the selecting operation on an oral model data, a scanning operation of scanning a processed actual structure to convert the scanned structure into a three-dimensional scan model, and an aligning operation of connecting an overlapping part between the three-dimensional library model and the three-dimensional scan data to be combined to each other.

In addition, the aligning operation may align the three-dimensional library model and the three-dimensional scan model to be combined to each other using an iterative closet point (ICP) algorithm.

In addition, the selecting operation may set a target position at which the three-dimensional library model is to be installed on the oral model data.

In addition, the library model setting operation may dispose the three-dimensional library model on an arbitrary point on the oral model data.

In addition, the library model setting operation may automatically dispose the three-dimensional library model selected in the selecting operation at the set target position.

In addition, when the scanning operation is performed, data from an upper portion of the three-dimensional library model by a certain height may be deleted.

In addition, the height is adjustable according to the user's operation.

In addition, the method may further include a comparing operation of comparing data of the three-dimensional scan model acquired in the scanning operation with data of the three-dimensional library model.

In addition, in the aligning process between the data of the three-dimensional scan model and the data of the three-dimensional library model, data from a part where the data do not overlap each other up to the upper portion of the three-dimensional library model may be deleted.

In addition, whether the data of the three-dimensional scan model and the data of the three-dimensional library model overlap each other may be determined depending on a data density of the data of the three-dimensional scan model.

In addition, the three-dimensional library model may be aligned with the three-dimensional scan model formed at the target position corresponding to the three-dimensional scan model acquired in the scanning operation, and the three-dimensional library model may be disposed.

Meanwhile, a method for matching structure data according to another embodiment of the present disclosure may include a selecting operation of selecting a three-dimensional library model from a library before a structure within an oral cavity is processed, and an aligning operation of generating a three-dimensional structure model by merging the three-dimensional library model with a three-dimensional scan model after the structure is processed.

In addition, the aligning operation may use a part of the three-dimensional library model as a first area of the three-dimensional structure model, and use a part of the three-dimensional scan model as a second area of the three-dimensional structure model.

In addition, the method may further include a library model setting operation of disposing the three-dimensional library model selected in the selecting operation at a position of the three-dimensional scan model in the three-dimensional oral model including the three-dimensional scan model.

In addition, in the library model setting operation, an iterative closet point (ICP) algorithm may be used for the arrangement of the three-dimensional library model may use.

In addition, after the library model setting operation, remaining parts other than a first area application part in the three-dimensional library model may be deleted.

Meanwhile, a system for matching structure data according to the present disclosure may include a storage unit configured to store a three-dimensional library model before a structure within an oral cavity is processed, a scan unit configured to acquire a three-dimensional scan model after the structure is processed, and a calculation unit configured to generate a three-dimensional structure model by merging the three-dimensional library model with the three-dimensional scan model.

In addition, a first area of the three-dimensional structure model may be a first area application part of the three-dimensional library model, and a second area of the three-dimensional structure model may be a second area application part of the three-dimensional scan model.

In addition, the second area of the three-dimensional structure model may include a processed surface of the structure.

In addition, the first area of the three-dimensional structure model may be an area from a lower portion of the three-dimensional structure model up to a certain height.

In addition, a height of the first area may be a point spaced apart from the processed surface of the structure by a certain distance or more.

In addition, remaining parts other than the first area application part in the three-dimensional library model may be deleted.

In addition, remaining parts other than the second area application part in the three-dimensional scan model may be deleted, or substituted with the first area application part of the three-dimensional library model.

In addition, the calculation unit may dispose the three-dimensional library model at a position of the three-dimensional scan model in the three-dimensional oral model including the three-dimensional scan model, and merge the three-dimensional library model with the three-dimensional scan model.

In addition, the three-dimensional oral model may be acquired by scanning the inside of an oral cavity in which the structure is implanted or the inside of the oral cavity before the structure is implanted.

### [Advantageous Effects of Invention]

It is possible to actually implement, as the digital data, the precise shape to which the structure to be used is applied through the prep using the method for matching the structure data according to the present disclosure.

In addition, the pre-equipped three-dimensional library model data is used for the data under the margin line with high scan difficulty upon three-dimensional scan realistically in the three-dimensional structure model data, so that it is possible to have the data for the portion for which the three-dimensional scan is difficult, and acquire the more complete oral inside model data of the patient.

### [Brief Description of Drawings]

FIG. 1 is a view referred to describe a configuration of an implant prosthesis including a structure used in a method for matching structure data according to the present disclosure.
FIG. 2 shows a mock-up of a structure equipped as three-dimensional library model data in the method for matching the structure data according to the present disclosure.
FIG. 3 is a view showing that the structure is not processed and inserted into an oral model in the method for matching the structure data according to the present disclosure.
FIG. 4 is a view showing that a part of an upper end of the structure inserted into the oral model is processed to be removed in the method for matching the structure data according to the present disclosure.
FIG. 5 shows the processed structure inserted into a maxilla of any patient's oral cavity in the method for matching the structure data according to the present disclosure.
FIG. 6 is a schematic flowchart of the method for matching the structure data according to the present disclosure.
FIG. 7 shows a screen of selecting the three-dimensional library model embedded in a library on a user interface screen using the method for matching the structure data according to the present disclosure.
FIG. 8 is a view showing the setting of a range of a height at which the three-dimensional library model data is to be used on the user interface screen using the method for matching the structure data according to the present disclosure.
FIG. 9 is a view schematically showing a process of overlapping the three-dimensional library model data of the structure and the three-dimensional scan model data, and removing three-dimensional library model data with respect to a portion where the three-dimensional scan model data is input in the method for matching the structure data according to the present disclosure.
FIG. 10 is a view showing that a three-dimensional structure model data obtained by combining and merging the three-dimensional library model data and three-dimensional scan model data of the structure is formed on the three-dimensional oral model data in the user interface screen using the method for matching the structure data according to the present disclosure.
FIG. 11 is a view for describing a system for matching structure data within an oral cavity according to the present disclosure.

### [Description of Embodiments]

Hereinafter, some embodiments of the present disclosure will be described in detail with reference to exemplary drawings. In denoting reference numerals to the components of each drawing, it should be noted that the same reference numerals are denoted by the same components as much as possible even though the same components are shown in different drawings. In addition, in describing embodiments of the present disclosure, when it is determined that a detailed description of a related known configuration or function obscures the understanding of the embodiments of the present disclosure, the detailed description thereof will be omitted.

In describing the components of the embodiments of the present disclosure, terms such as first, second, A, B, (a), and (b) may be used. These terms are only to distinguish the components from other components, and the essence, sequence, order, or the like of the corresponding component is not limited by the terms. In addition, unless otherwise defined, all terms used herein, including technical or scientific terms, have the same meaning as commonly understood by those skilled in the art to which the present disclosure pertains. Terms such as those defined in a commonly used dictionary should be interpreted as having a meaning consistent with the meaning in the context of the related art, and are not be interpreted in an ideal or excessively formal meaning unless explicitly defined in the present application.

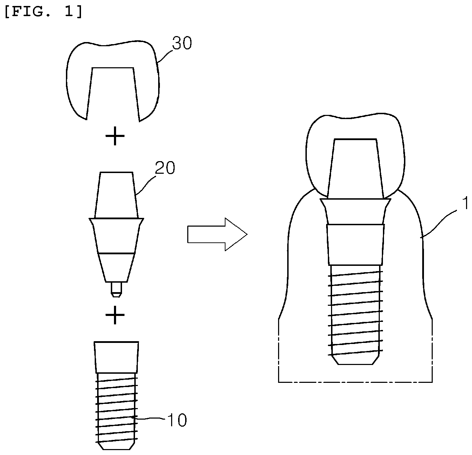

FIG. 1 is a view referred to describe a configuration of an implant prosthesis including a structure used in a method for matching structure data according to the present disclosure.

Referring to FIG. 1, a dental implant crown procedure refers to a procedure that provides a substitute for a damaged tooth when a tooth is lost by removing (extracting) the damaged tooth in the patient's oral cavity, which refers to implanting artificially manufactured teeth in order to substitute natural teeth (since temporary teeth are separated in childhood, the natural teeth mentioned in this specification may be normally permanent teeth).

A dental implant crown may be largely classified into three main components. One is a fixture 10 that is formed on an outer circumferential surface to have a shape of a rotating screw and rotated and fixed to a gingival 1. The fixture 10 is a structure corresponding to the root of a tooth in a natural tooth, and may be firmly fixed to an alveolar bone. In addition, the fixture 10 may form a cavity in an inward direction of a direction opposite to the direction fixed toward the gingival 1. The cavity formed in the fixture 10 may be coupled to an object having a protrusion corresponding thereto.

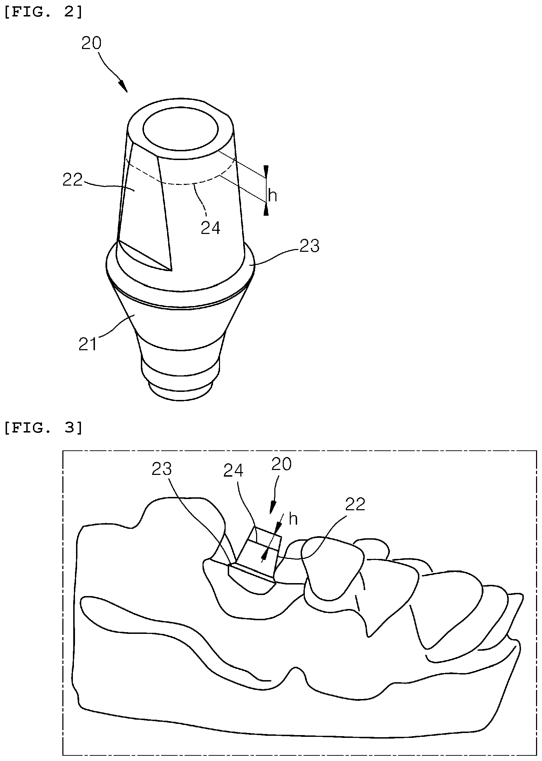

FIG. 2 shows a mock-up of a structure 20 of equipped as three-dimensional library model data in the method for matching the structure data according to the present disclosure.

Referring to FIG. 2, one end of the structure 20 may be fitted into and coupled to the cavity of the fixture 10. An outer circumferential surface of one end of the structure 20 fitted into and coupled to the cavity of the fixture 10 may be formed to have the same shape as an inner circumferential surface of the cavity of the fixture 10. Meanwhile, the structure 20 may increase in diameter from one end fitted into and coupled to the cavity of the fixture 10 toward the other end. This allows the structure 20 to be more closely coupled to the gingival. As described above, a portion whose diameter increases from one end in a longitudinal direction is called a cuff part 21, and the cuff part 21 may be selected and used by comprehensively considering the patient's gingival height (G/H) and the size of a space left on the gingival after tooth extraction. Meanwhile, in the present disclosure, the structure 20 may be an abutment supported on the gum for crown treatment.

In addition, the structure 20 further includes a margin line part 23 that is formed continuously with the cuff part 21, and decreases in diameter in one direction of a length. The margin line may be a portion where the structure is exposed from the gingival, and the portion functions as a key factor when the crown is put on thereafter. A post part 22 may be formed to extend in one direction of the length continuously with the margin line part 23 again. For example, the post part 22 may have a cylindrical shape, and the diameter of the post part 22 may be smaller than the diameter of the margin line connected to the cuff part 21. However, the post part 22 may not have a perfect cylindrical shape, but may also have a truncated cone shape in which the diameter of the cross section decreases from a lower portion to an upper portion of the post part 22. Any shape of the post part 22 may also be applied as long as the crown 30 to be described later may be easily coupled and the shape of the post part 22 is suitable for the inside of the patient's oral cavity.

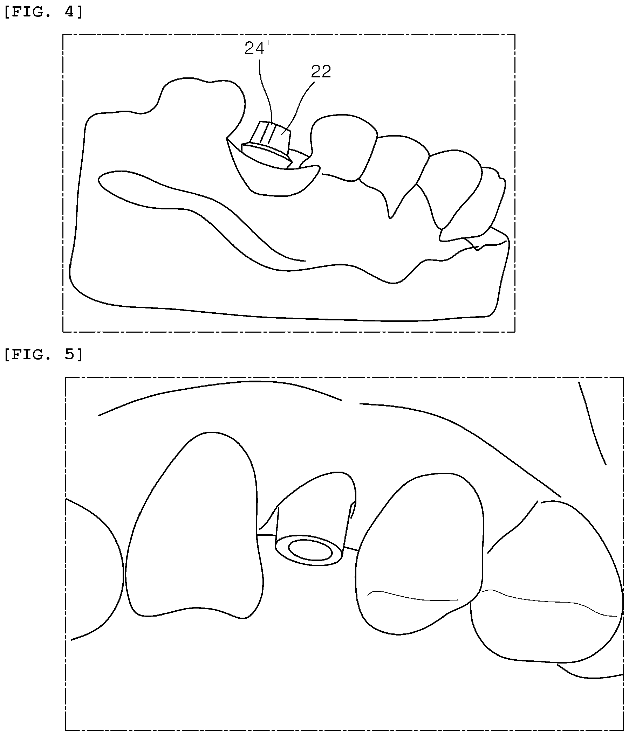

FIG. 3 is a view showing that the structure is not processed and inserted into an oral model in the method for matching the structure data according to the present disclosure, FIG. 4 is a view showing that a part of an upper end of the structure inserted into the oral model is processed to be removed in the method for matching the structure data according to the present disclosure, and FIG. 5 shows the processed structure inserted into a maxilla of any patient's oral cavity in the method for matching the structure data according to the present disclosure. Referring to FIGS. 3 to 5, the structure 20 may also use off-the-shelf products manufactured by a number of companies that manufacture the existing prosthetic treatment products, and also use custom products through making orders. Although many off-the-shelf products are adopted and used in the prosthetic treatment field, it is practically impossible to use the off-the-shelf product as it is due to the characteristics of each individual having different oral cavity structures. Accordingly, even when the off-the-shelf structure products are applied to the patient's oral cavity, it is necessary to process the tooth at a position where the patient wants to take the crown treatment (where the previously damaged tooth is positioned) to be formed harmoniously with the shapes, heights, and the like of adjacent teeth and teeth shapes. The operation process is referred to as a preparation process, and the off-the-shelf structure product may be applied to the inside of the patient's oral cavity after the height of the structure 20 and the coupling surface to be coupled to the crown 30 are processed through the preparation process.

After the off-the-shelf product is applied to the oral model of the patient or the inside of the actual patient's oral cavity by performing the above-described preparation process, the cuff part 21 corresponding to the lower portion of the margin line is inserted into the gingival and supported by the gingival tissue, so that it is difficult to acquire scan data through a three-dimensional scanner. Meanwhile, since the preparation process is performed for the post part 22 corresponding to the upper portion of the margin line part 23, the cuff part 21 is not worn out unless there is a special circumstance after the initial implant and crown procedures.

Accordingly, for the portion where it is difficult to acquire scan data through the three-dimensional scanner, model data of the patient's entire oral cavity may be completed by using three-dimensional modeled data of the structure designed and manufactured according to the specified standard.

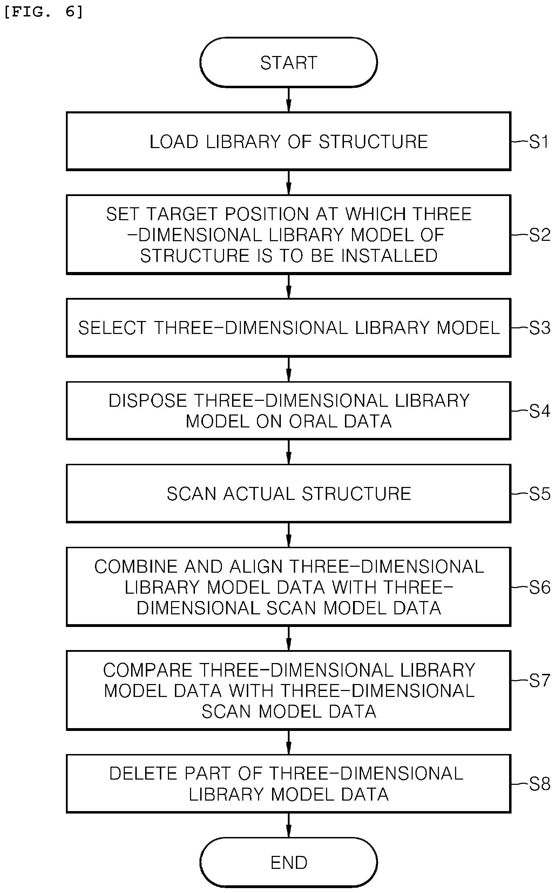

FIG. 6 is a schematic flowchart of the method for matching the structure data according to the present disclosure.

Referring to FIG. 6, the method for matching structure data according to the present disclosure may first include a library loading operation (S1) of loading a library for the structure in the user interface. To perform the observation and analysis on the three-dimensional oral model data, the three-dimensional oral model data should be expressed on a display. The three-dimensional oral model data may be displayed on the user interface (UI) of the display device, and the user interface may variously include a PC application program, a mobile application, a web application, and the like. The user interface has built-in data on the off-the-shelf structure conventionally sold and supplied in prosthetic treatment field. The built-in structure data may be three-dimensional modeled data (hereinafter, referred to as three-dimensional library model data), and the three-dimensional library model data may be in STP and STL file formats commonly used.

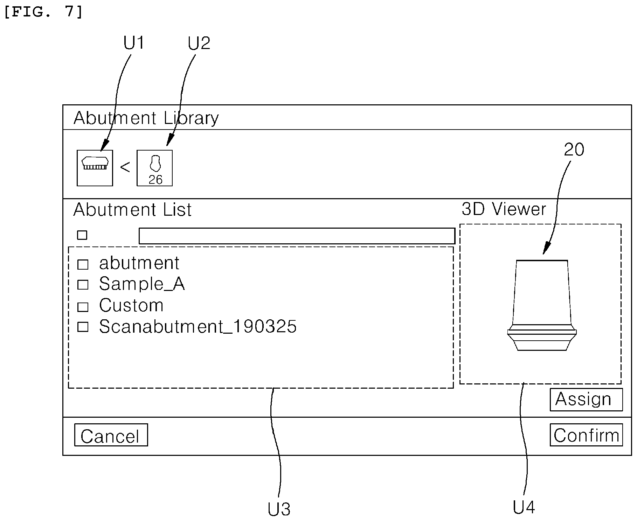

FIG. 7 shows a screen of selecting the three-dimensional library model embedded in a library on a user interface screen using the method for matching the structure data according to the present disclosure.

Referring to FIGS. 6 and 7, the method for matching structure data according to the present disclosure may further include an operation of setting a target position at which the three-dimensional library model of the structure is to be installed on the three-dimensional oral model data (S2) in order to dispose the structure 20 on the three-dimensional oral model data on the user interface. At this time, the target position may be a position of a tooth preset on the user interface, and the position of the tooth may be numbered according to the disposed position. The target position may be selected by the user on the user interface.

The method for matching the structure data according to the present disclosure may include a selecting operation (S3) of loading the library for the structure and then selecting the three-dimensional library model of the structure 20 by the user. The three-dimensional library model of the structure 20 may selectively display models matching the target position in the above-described operation. For example, when the target position corresponding to a molar in the above-described process, models of the structure 20 applicable to the molar position may be selectively displayed, and when the target position corresponding to a canine is selected, three-dimensional library models of the structure 20 applicable to the canine position may be selectively displayed. By selectively displaying the three-dimensional library models of the structure 20 applicable according to the target position, the user may quickly select the three-dimensional library model of the structure 20 suitable for the patient's oral cavity without searching for the entire library of the structure, so that it is possible to acquire efficient three-dimensional oral model data.

In the selection as described above, a position where the damaged tooth part (i.e., the part to which the structure is to be applied) is positioned in a maxilla and mandible selection unit U1 is first selected. At this time, the user may select the maxilla or the mandible from the maxilla and mandible selection unit U1, and when the maxilla and the mandible are selected, a target position selection unit U2 capable of selecting the tooth disposed in each arch is activated to select the tooth of the target position. At this time, the target positions may be displayed in a predetermined order, and the predetermined order may be tooth numbers corresponding to the target positions according to a universal numbering system. When the target position is completely selected, the three-dimensional library models of the structure 20 suitable to be applied to the corresponding target position selected by the model selection unit U3 are displayed. Since only the models of the structure 20 suitable to be applied to the corresponding target position are displayed, the user may quickly find and select the necessary model. Meanwhile, when the necessary three-dimensional library model of the structure 20 is selected, a schematic model of the three-dimensional model data is displayed in a model preview U4 window at the right, so that it is possible to confirm whether the three-dimensional library model of the structure 20 selected by the user has been accurately selected.

Meanwhile, when the model of the structure 20 suitable to be applied to the patient in the selecting operation (S3), the corresponding model is disposed on the three-dimensional oral model data (the library model setting operation (S4)). At this time, the three-dimensional library model of the structure 20 may also be disposed at any position on the three-dimensional oral model data, or may be automatically disposed at a set target position. In addition, the three-dimensional library model of the structure 20 may be disposed at any position of the user interface where the three-dimensional oral model data is displayed, and disposed to be spaced apart from the three-dimensional oral model data. Meanwhile, in order for the three-dimensional library model of the structure 20 to be automatically disposed at the target position, the selected arrangement position of the structure 20 may be determined by comprehensively considering the depth at which the structure 20 is actually inserted into the gingival, the distance from adjacent tooth data, and the like.

When the three-dimensional library model of the structure 20 is selected from the structure library and completely disposed on the oral model data (the arrangement at this time may be the arrangement at an arbitrary position in a space where the oral model data is displayed, or automatically at the target position) may be arranged), and the method for matching the structure data may further include a scanning operation (S5) of converting a scanned actual structure into three-dimensional scan data by scanning the actual structure in order to acquire data on the prepared structure to be actually applied to the inside of the patient's oral cavity. The scanning operation (S5) may capture the prepared actual structure, and the three-dimensional scanner may generate three-dimensional volume data from the acquired image data. At this time, the three-dimensional volume data may be three-dimensional voxel data including brightness information or the like on a point or a surface.

When the actual structure is captured in the scanning operation (S5), a part in which the same data as the three-dimensional library model data of the structure stored in the library is acquired, and a part in which the deformed data is acquired from the off-the-shelf structure model due to the preparation process may be generated. At this time, there is a part that requires the three-dimensional scan data of the actual structure above a specific reference position, and requires the three-dimensional library model data of the structure stored in the library below the specific reference position. Accordingly, data may be selectively selected to apply the structure 20 to the three-dimensional oral model data.

Accordingly, the method for matching the structure data according to the present disclosure may further include an aligning operation (S6) of connecting overlapping parts so that the three-dimensional library model of the structure 20 and three-dimensional scan model data SD are combined with each other. The alignment may also mean the connection between the data acquired through three-dimensional scanning, and also mean that the three-dimensional scan model data SD acquired through the three-dimensional scanning and three-dimensional library model data LD of the structure stored in the library are correspondingly connected to each other. In the case of a part where the three-dimensional scan model data SD acquired through the three-dimensional scanning is determined to be the same as the three-dimensional library model data LD of the structure 20 stored in the library and is connected thereto, the part is not a prepared part, so that the three-dimensional library model data LD of the structure loaded through the library is more accurate. On the other hand, when it is determined that the three-dimensional scan model data SD is different from the three-dimensional library model data LD of the structure stored in the library, the part may be a prepared part, so that it is preferable that the three-dimensional scan model data SD corresponding to an actual structure is used. Meanwhile, in the aligning operation, the three-dimensional library model data LD loaded through the library and the three-dimensional scan model data SD may be aligned by using an iterative closet point (ICP) algorithm to be combined and connected to each other. Data may be quickly aligned through the point connection by using the ICP algorithm.

Meanwhile, when the three-dimensional library model data LD and the three-dimensional scan model data SD are aligned and combined through the aligning operation (S6), data of a certain height h may be deleted from the upper portion of the three-dimensional library model of the structure 20 on the user interface. At this time, the part of the model left as the three-dimensional library model data may be referred to as a first area application part. The merged three-dimensional structure 20 model may appear by the alignment and combination, and the deletion of data for the specific area. When the certain height h is deleted from the upper portion of the three-dimensional structure 20 model, the three-dimensional library model data LD of the structure loaded from the library appears on the lower portion of the three-dimensional structure 20 model, and the three-dimensional scan model data SD acquired by scanning the actual structure appears on the upper portion of the three-dimensional structure model. As described above, the lower portion (first area) of the three-dimensional structure model uses the first area application part among the three-dimensional library model data LD of the structure loaded from the library, and the upper portion (second area) of the three-dimensional structure model uses a second area application part among the three-dimensional scan model data SD of the actual structure, so that it is possible to acquire data similar to the actual structure to be applied to the patient. In addition, it is possible to improve the reliability of the oral model data using the library data (LD) for a part where the scan may not be easily performed. At this time, the lower portion (first area) of the three-dimensional structure 20 model includes a part that is inserted and fixed into the gingival, and the upper portion (second area) of the three-dimensional structure model means a part protruding to the outside of the gingival to be exposed into the oral cavity and prepared, and the upper and lower portions may be referred to regardless of the maxillary/mandibular positions, and the lower portion of the structure does not necessarily refer to the mandible or downward direction with respect to the patient's oral cavity.

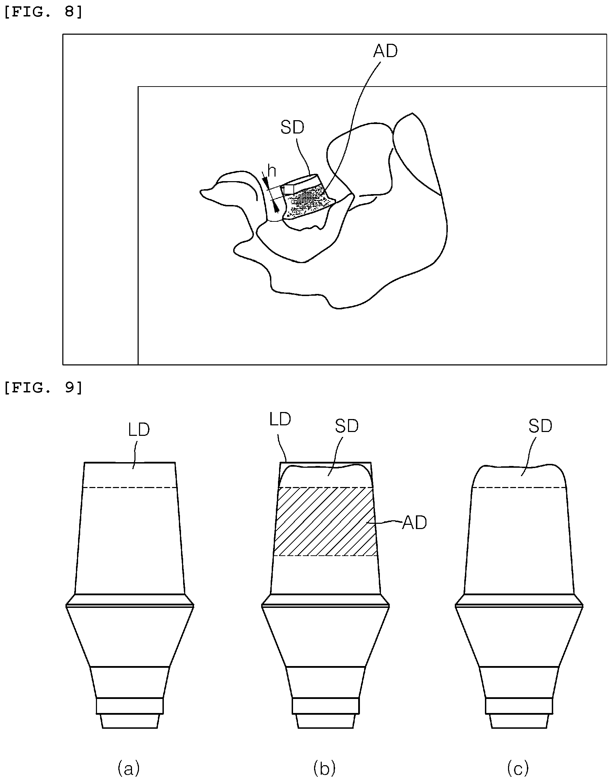

FIG. 8 is a view showing the setting of a range of a height at which the three-dimensional library model data is to be used on the user interface screen using the method for matching the structure data according to the present disclosure. Referring to FIG. 8, the certain height h deleted from the upper portion of the three-dimensional library model of the structure may be a value preset on the user interface. However, the height to be deleted may also be adjusted according to the user's subjective operation. In other words, the user may also directly designate the range in which the modeling data LD of the three-dimensional library model of the structure built in the library is to be used. By adjusting the upper height h of the structure model to be deleted by the user, it is possible to acquire data more suitable for the user.

Meanwhile, the method for matching the structure data according to the present disclosure may further include a comparing operation (S7) of comparing the three-dimensional scan model data SD acquired in the scanning operation (S5) with the three-dimensional library model data LD of the structure. In the comparing operation (S7), the processor connected to the three-dimensional scanner determines whether the three-dimensional library model data LD of the structure loaded from the library and the three-dimensionally scanned three-dimensional scan model data SD match with each other. When the three-dimensional library model data LD of the structure loaded from the library and the three-dimensional scan model data SD match with each other, the three-dimensional scan model data SD may be expressed on the user interface in the form of being overwritten on the library structure model data LD. At this time, the overwritten form may be displayed to have a color different from that of the disposed three-dimensional library model data LD of the structure without substituting data with the scan data SD.

Since the three-dimensional library model data LD loaded from the library and the three-dimensionally scanned three-dimensional scan model data SD do not match with each other from the part where the actual structure has been prepared, data mismatch occurs from the height or more of the specific structure. In the part where the data mismatch occurs, the alignment between the three-dimensional library model data LD of the structure loaded from the library and the three-dimensionally scanned three-dimensional scan model data SD does not occur, and the part where the alignment does not occur is recognized as the prepared part, so that it is necessary to delete the library structure data LD.

FIG. 9 is a view schematically showing a process of overlapping the three-dimensional library model data LD of the structure 20 and the three-dimensional scan model data SD, and removing three-dimensional library model data LD with respect to a portion where the three-dimensional scan model data SD is input in the method for matching the structure data according to the present disclosure.

(a) of FIG. 9 is a view showing the three-dimensional library model data LD of the structure equipped in the library. The post part 22 of the structure is formed to be long enough to use the off-the-shelf product as it is, and should enable the height thereof or a crown bonded surface 24' to be processed in a shape desired by the user through the preparation. Meanwhile, referring to (b) of FIG. 9, when the processed structure 20 is scanned, the prepared part is input to the three-dimensional scan model data SD through the three-dimensional scanner. When the structure data input to the three-dimensional scan model data SD is compared with the three-dimensional library model data LD of the structure, it may be confirmed that the three-dimensional scan model data SD is prepared to have an irregularity more than the three-dimensional library model data LD, and the height thereof is formed low. In addition, since the part not prepared and the three-dimensional library model data LD match with each other, they match with each other as the same data like the overlapping part shown in the drawing. The calculation unit may delete the three-dimensional library model data LD for the part corresponding to the upper portion of the overlapping part AD on the user interface, that is, where the three-dimensional library model data LD and the three-dimensional scan model data SD do not match with each other so that only the three-dimensional scan model data SD may remain. As a result, as shown in (c) of FIG. 9, the upper portion (second area) of the overlapping part AD remains as the second area application part in the three-dimensional scan model data SD. In addition, parts (first area) other than the three-dimensional scan model data SD remain in the three-dimensional library model data LD of the structure as the first area application part, so that it is possible to create new three-dimensional structure model data in which the three-dimensional scan model data SD and the three-dimensional library model data LD are combined with each other.

In the above-described process, the overlapping part AD may be determined depending on a data density in a process in which the three-dimensional library model data LD and the three-dimensional scan model data SD are aligned and overlapped with each other. As the actual structure is continuously scanned, the three-dimensional scan model data SD is accumulated, and the accumulated three-dimensional scan model data SD is aligned with the three-dimensional library model data LD of the structure equipped in the library. For the part where data is matched and aligned, whether data matches is continuously confirmed, and thus the part may be determined as the overlapping part AD.

Meanwhile, when the three-dimensional library model is disposed at an arbitrary position on the oral model data in the library model setting operation (S4), the library model data may not be automatically disposed at the target position just because the user sets the target position. At this time, in the scanning operation (S5), the three-dimensional scan model data SD may be acquired by scanning the actual structure 20, and matched with the three-dimensional library model data LD disposed at the arbitrary position by the comparing operation (S7) so that the three-dimensional library model data LD is disposed at the correct target position. In other words, by scanning the part where the structure is to be disposed on the three-dimensional oral model data and matching the three-dimensional library model data LD with the part where the three-dimensional scan model data SD is acquired, the three-dimensional library model data LD may be disposed at a suitable target position.

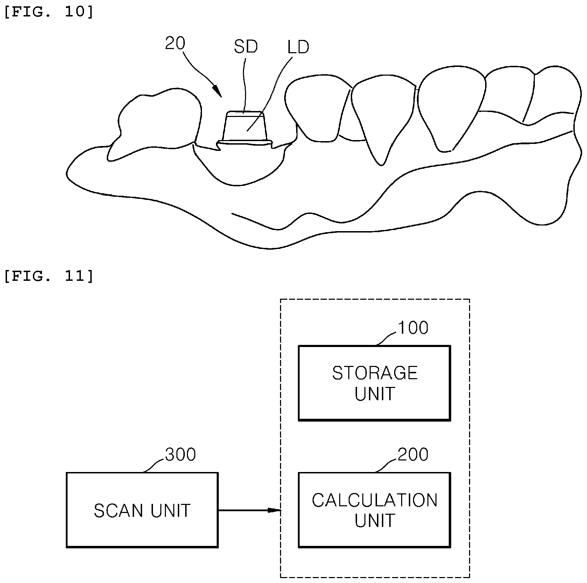

FIG. 10 is a view showing that a three-dimensional structure model data obtained by combining and merging the three-dimensional library model data LD and three-dimensional scan model data SD of the structure 20 is formed on the three-dimensional oral model data in the user interface screen using the method for matching the structure data according to the present disclosure. Referring to FIG. 10, according to the above-described method for matching the structure data, by combining the first area application part among the three-dimensional library model data LD equipped in the library of the lower portion (first area) of the three-dimensional structure 20 model with the second area application part among the three-dimensional scan model data SD of the prepared actual structure of the upper portion (second area) of the three-dimensional structure 20 model, it is possible to acquire the precise three-dimensional oral model data closer to the actual product and the part having high scan difficulty may obtain library data.

Hereinafter, a system for matching structure data within the oral cavity according to the present disclosure will be described in detail. In the description, the contents overlapping with the above description will be briefly described or omitted.

FIG. 11 is a view for describing a system for matching structure data within an oral cavity according to the present disclosure.

Referring to FIG. 11, the system for matching the structure data within the oral cavity according to the present disclosure may include a storage unit 100 configured to store the three-dimensional library model data before the structure 20 is processed, and a scan unit 300 configured to acquire the three-dimensional scan model that is a shape appearing after the actual structure is prepared by the user (or therapist). At this time, the scan unit 300 may be a table scanner configured to perform a scan by disposing a plaster acquired for the patient's oral cavity on a tray, or may also be a handheld intraoral scanner configured to perform the three-dimensional scan by the user directly drawing one end into or out from the patient's oral cavity and capturing the inside of the oral cavity. However, the scan unit 300 is not necessarily limited to the intraoral scanner, and any means capable of acquiring three-dimensional scan data for the patient's oral cavity may be used.

Meanwhile, when there is an off-the-shelf structure 20 product as the structure 20, the three-dimensional library model data for the corresponding product may be equipped in the library. At this time, the three-dimensional library model data may be a three-dimensional modeling format file such as STP or STL. The storage unit 100 and the scan unit 300 may load and transmit data on and to the same user interface, and the storage unit 100 and a calculation unit 200 may be included in the same device. For example, the storage unit 100 and the calculation unit 200 may also correspond to a storage medium and a processor such as a central processing unit, respectively, to configure a personal computer. The scan unit 300 may be communicatively connected to a device including the storage unit 100 and the calculation unit 200 to transmit the three-dimensional scan data SD acquired through the three-dimensional scan to the calculation unit 200. At this time, communicatively connecting may also be a wired connection between the scan unit 300 and the device via a specific terminal, or may also be the connection performed so that data may be transmitted through wireless communication.

As described above, when the actual structure is installed in the patient's oral cavity with respect to the off-the-shelf structure (the abutment connecting the fixture and the crown may correspond to the structure), the scanning difficulty of the part supported by the gingival increases, so that it is possible to improve the reliability of the three-dimensional oral model data using the model data built in the previously existing library rather than the data obtained by the scan. In addition, when the library model data of the off-the-shelf structure is used for the prepared part (herein, the part prepared and deformed as compared to the off-the-shelf product is referred to as the second area), rather, the dental prosthesis customized for the patient may not be provided, so that the part preferably uses the data scanned by the three-dimensional scanner. In other words, the second area may include a prepared processed surface of the actual structure.

Commercially available off-the-shelf structures are manufactured with the possibility of such preparation in mind, and thus the commercially available off-the-shelf structures are designed and sold to have a height higher than the height applied to the patient's oral cavity. The user cuts a part of the structure and bends the surface of the structure, so that it is possible to improve bondability with the crown to be applied to the structure and uniformly match the height with adjacent teeth.

Referring to FIGS. 2 and 8, parts of the cuff part 21, the margin line part 23, and the post part 22 based on the surface corresponding to the lowermost end of the cuff part 21 is subjected to a special processing process, and thus may be expressed on the user interface using the three-dimensional library model data equipped in the library. The part may be referred to as the first area, and the first area refers to an area from the lowermost end of the cuff part (i.e., the lower portion of the structure) to a certain height.

Referring to FIG. 9, the height of the first area may be a point spaced apart from the processed surface of the structure by a certain distance or more. In (a) of FIG. 9, the preparation may be performed up to a point separated by a certain distance, that is, a certain height h from an upper surface of the existing structure before processed. Thereafter, as shown in (c) of FIG. 9, the preparation may be performed up to the range required by the user, and the existing off-the-shelf structure is used for the remaining area. At this time, the non-prepared area may be referred to as the first area, and the prepared area may be referred to as the second area.

Meanwhile, the system for matching the structure data within the oral cavity according to the present disclosure may further include the calculation unit 200 configured to generate the three-dimensional structure model by merging the three-dimensional library model and the three-dimensional scan model. The calculation unit 200 may appropriately select and merge data with respect to the three-dimensional library model data stored in the storage unit 100 and the three-dimensional scan model data input from the scan unit 300.

When the user loads the three-dimensional library model data LD stored in advance in the storage unit 100 on the user interface and scans the inside of the patient's oral cavity to which the actual structure is applied through the scan unit 300, the three-dimensional scan model data SD is input. The calculation unit 200 compares the existing three-dimensional library model data with the three-dimensional scan model data SD. In the comparison process, the three-dimensional library model data LD loaded from the storage unit 100 for the certain height (the first area) from the lower portion of the structure including the overlapping part AD as it is based on the part corresponding to the overlapping part AD. At this time, data for the remaining parts other than the first area application part in the three-dimensional library model data LD may be deleted.

In addition, even for the three-dimensional scan model data SD, the scan model data may be maintained for the part to which the second area is applied, that is, the area that is prepared and deformed from the off-the-shelf product. Meanwhile, since the scan data corresponding to the lower portion (first area) of the structure from the part corresponding to the overlapping part AD is more inaccurate than the first area application part among the existing three-dimensional library model data LD, the three-dimensional scan model data SD may be operated to be maintained for only the second area application part and deleted or substituted with the three-dimensional library model data LD for the remaining parts.

Meanwhile, the calculation unit 200 matches the three-dimensional library model data LD to be set at the target position in the three-dimensional oral model including the three-dimensional scan model, and merges the three-dimensional scan model data SD into the part of the three-dimensional library model data LD so that the three-dimensional scan model data SD reflects data for the actually prepared structure At this time, as described above, by selecting the first area application part among the three-dimensional library model data LD of the first area of the three-dimensional structure model data and the second area application part among the three-dimensional scan model data SD of the second area thereof, respectively, and merging the first and second area application parts, it is possible to acquire the precise data corresponding to the actually prepared structure, thereby providing the prosthetic treatment product of the precise standard to the patient. Meanwhile, the above-described three-dimensional oral model data may also be acquired by scanning the inside of the patient's oral cavity in which the actual structure is implanted, and may also be acquired by scanning the inside of the patient's oral cavity before the structure is implanted.

The above description is merely illustrative of the technical spirit of the present disclosure, and various modifications and changes will be possible by those skilled in the art to which the present disclosure pertains without departing from the essential characteristics of the present disclosure.

Accordingly, the embodiments disclosed in the present disclosure are not intended to limit the technical spirit of the present disclosure but to describe it, and the scope of the technical spirit of the present disclosure is not limited by these embodiments. The scope of the present disclosure should be construed by the appended claims, and all technical spirits within the scope equivalent thereto should be construed as being included in the scope of the present disclosure.

### [Industrial Applicability]

The present disclosure provides a method for merging the three-dimensional scan model and three-dimensional library model of the structure in order to improve reliability of the three-dimensional model of the structure and the system using the same.

## Claims

1. A method for matching structure data, the method comprising:
a library loading operation of loading a library of a structure on a user interface;
a selecting operation of selecting a three-dimensional library model from the loading operation by a user;
a library model setting operation of disposing the three-dimensional library model selected in the selecting operation on an oral model data;
a scanning operation of scanning a processed actual structure to convert the scanned structure into a three-dimensional scan model; and
an aligning operation of connecting an overlapping part between the three-dimensional library model and the three-dimensional scan data to be combined to each other.

2. The method of claim 1,
wherein the aligning operation aligns the three-dimensional library model and the three-dimensional scan model to be combined to each other using an iterative closet point (ICP) algorithm.

3. The method of claim 1,
wherein the selecting operation sets a target position at which the three-dimensional library model is to be installed on the oral model data.

4. The method of claim 3,
wherein the library model setting operation disposes the three-dimensional library model on an arbitrary point on the oral model data.

5. The method of claim 3,
wherein the library model setting operation automatically disposes the three-dimensional library model selected in the selecting operation at the set target position.

6. The method of any one of claim 4 or 5,
wherein when the scanning operation is performed, data from an upper portion of the three-dimensional library model by a certain height is deleted.

7. The method of claim 6,
wherein the height is adjustable according to the user's operation.

8. The method of claim 6, further comprising: a comparing operation of comparing data of the three-dimensional scan model acquired in the scanning operation with data of the three-dimensional library model.

9. The method of claim 8,
wherein in the aligning process between the data of the three-dimensional scan model and the data of the three-dimensional library model, data from a part where the data do not overlap each other up to the upper portion of the three-dimensional library model is deleted.

10. The method of claim 9,
wherein whether the data of the three-dimensional scan model and the data of the three-dimensional library model overlap each other is determined depending on a data density of the data of the three-dimensional scan model.

11. The method of claim 4,
wherein the three-dimensional library model is aligned with the three-dimensional scan model formed at the target position corresponding to the three-dimensional scan model acquired in the scanning operation, and the three-dimensional library model is disposed.

12. A method for matching structure data, the method comprising:
a selecting operation of selecting a three-dimensional library model which corresponds to a structure before processed within an oral cavity; and
an aligning operation of generating a three-dimensional structure model by merging the three-dimensional library model with a three-dimensional scan model which corresponds to a structure after processed.

13. The method of claim 12,
wherein the aligning operation uses a part of the three-dimensional library model as a first area of the three-dimensional structure model, and uses a part of the three-dimensional scan model as a second area of the three-dimensional structure model.

14. The method of claim 13, further comprising: a library model setting operation of disposing the three-dimensional library model selected in the selecting operation at a position of the three-dimensional scan model in the three-dimensional oral model including the three-dimensional scan model.

15. The method of claim 14,
wherein in the library model setting operation,
an iterative closet point (ICP) algorithm is used for the arrangement of the three-dimensional library model.

16. The method of claim 14,
wherein after the library model setting operation, remaining parts other than a first area application part in the three-dimensional library model are deleted.

17. A system for matching structure data comprising:
a storage unit configured to store a three-dimensional library model before a structure within an oral cavity is processed;
a scan unit configured to acquire a three-dimensional scan model after the structure is processed; and
a calculation unit configured to generate a three-dimensional structure model by merging the three-dimensional library model with the three-dimensional scan model.

18. The system for matching the structure data of claim 17,
wherein a first area of the three-dimensional structure model is a first area application part of the three-dimensional library model, and a second area of the three-dimensional structure model is a second area application part of the three-dimensional scan model.

19. The system for matching the structure data of claim 18,
wherein the second area of the three-dimensional structure model includes a processed surface of the structure.

20. The system for matching the structure data of claim 19,
wherein the first area of the three-dimensional structure model is an area from a lower portion of the three-dimensional structure model up to a certain height.

21. The system for matching the structure data of claim 20,
wherein a height of the first area is a point spaced apart from the processed surface of the structure by a certain distance or more.

22. The system for matching the structure data of claim 18,
wherein remaining parts other than the first area application part in the three-dimensional library model are deleted.

23. The system for matching the structure data of claim 18,
wherein remaining parts other than the second area application part in the three-dimensional scan model are deleted, or substituted with the first area application part of the three-dimensional library model.

24. The system for matching the structure data of claim 17,
wherein the calculation unit disposes the three-dimensional library model at a position of the three-dimensional scan model in the three-dimensional oral model including the three-dimensional scan model, and merges the three-dimensional library model with the three-dimensional scan model.

25. The system for matching the structure data of claim 24,
wherein the three-dimensional oral model is acquired by scanning the inside of an oral cavity in which the structure is implanted or the inside of the oral cavity before the structure is implanted.
